# EUROPEAN PATENT APPLICATION

(11) **EP 3 542 702 A1**
(43) Date of publication of application: **25.09.2019**
(21) Application number: 17872866.3
(22) Date of filing: 03.08.2017
(51) Int. Cl.: A61B 1/06, A61B 1/273, A61B 5/07

(54) **CAPSULE GASTROSCOPE MAGNETIC CONTROL SYSTEM**

(30) Priority: 15.11.2016 CN 201611036647; 02.12.2016 CN 201611099481
(71) Applicant: Shenzhen Jifu Technology Co., Ltd, Shenzhen, Guangdong 518055 (CN)
(72) Inventor: DENG, Wenjun, Shenzhen Guangdong518055 (CN); WANG, Jianping, Shenzhen Guangdong 518055 (CN); LIU, Shaoping, Shenzhen Guangdong 518055 (CN); SUN, Renhui, Shenzhen Guangdong 518055 (CN)
(74) Representative: J A Kemp
(86) International application number: PCT/CN2017/095761
(87) International publication number: WO 2018/090671

(57) **Abstract**

Provided is a capsule gastroscope magnetic control system comprising a rack (1) and a magnetic control device arranged on the rack (1). The magnetic control device comprises a magnet (2), a position control unit (3) and a magnetic line direction regulating unit (4). The position control unit (3) is arranged on the rack (1) so as to enable the magnet (2) to move on a plane which forms an angle of 90 ± 30 degrees with the horizontal plane; the magnetic line direction regulating unit (4) is arranged on the position control unit (3) for adjusting the direction of the magnetic line of the magnet (2).

## Description

### TECHNICAL FIELD

The disclosure relates to a field of medical devices, and more particularly to a capsule gastroscope magnetic control system.

### BACKGROUND

Nowadays, under the social pressure and unhealthy diet, the digestive system of modern people is prone to problems and causes considerable trouble. Therefore, the health of digestive system has become one of the focuses of modern people.

When a physician needs to perform further examination on the stomach in the digestive system to confirm the symptom, it is necessary to extend a gastroscope with one end provided with a camera and an illumination device and the other end with a long tube connecting device into the stomach from the mouth of the subject. Such measures will cause great pain to the subject, such that the subject will have a sense of fear and resistance to gastroscopy.

In order to improve above-mentioned technology, a gastric examination method using a capsule gastroscope as a stomach detection device has emerged. A human body only needs to lie on a platform, and a magnetic control system located above the human body may adjust and control the position of the capsule gastroscope with camera and illumination devices swallowed into a stomach of a subject in advance, such that the observation angle can be changed, and the pain of the subject can be greatly reduced.

However, when the human body lies down, the stomach is deformed by the action of gravity, such that the space of the stomach cavity is greatly reduced. The reduced space not only renders a bad control effect of the magnetic control system on the capsule gastroscope, but also affects the photographing effect of the capsule gastroscope. It is necessary to control the movement of the capsule gastroscope by a magnetic control system for many times in order to get a complete photograph of the stomach environment, such that the shooting time is longer and the image presentation effect is poorer.

### SUMMARY

The embodiments of the disclosure provide a capsule gastroscope magnetic control system, which aims to address the problems of the existing lying capsule gastroscope magnetic control system of long shooting time and poor image performance.

The embodiments of the disclosure are implemented by a capsule gastroscope magnetic control system including:
a rack; and
a magnetic control device located on the rack, the magnetic control device including:
a magnet;
a position control device located on the rack and capable of moving the magnet on a plane forming an angle of 90 ± 30 degrees with a horizontal plane; and
a magnetic line direction adjusting device being located on the position control device and configured to adjust a magnetic line direction of the magnet.

In the embodiment of the disclosure, the subject stands upright in front of the capsule gastroscope system after swallowing the capsule gastroscope with the camera and the illumination device into the stomach in advance. The detector can control the movement of the magnet in the capsule gastroscope magnetic control system through the position control device on the plane forming an angle of 90 ± 30 degrees with the horizontal plane. The magnetic line direction adjustment device controls the movement of the capsule gastroscope in the stomach of the subject to obtain images of the stomach. By designing the magnetic control system with an examination control mode of upright standing, the cavity of the stomach of the subject can be kept in a relatively complete state, such that the magnetic control system can more easily control the capsule gastroscope. The image efficiency and image effect of the capsule gastroscope can be greatly improved.

### BRIEF DESCRIPTION OF THE DRAWINGS

FIG. 1 is a schematic diagram of a capsule gastroscope magnetic control system and a subject according to an embodiment of the disclosure.
FIG. 2 is a schematic diagram of a structure of capsule gastroscope magnetic control system according to an embodiment of the disclosure.
FIG. 3 is schematic diagram of a structure of a magnetic line direction adjusting device according to an embodiment of the disclosure.

### DETAILED DESCRIPTION OF THE EMBODIMENTS

The present disclosure will be described in details in combination with the accompanying drawings and embodiments such that the technical solution and advantages of the present disclosure will be more apparent. It should be understood that the particular embodiments are described for the purpose of illustrating rather than restricting the present disclosure.

In the embodiment of the disclosure, the subject stands upright in front of the capsule gastroscope system after swallowing the capsule gastroscope with the camera and the illumination device into the stomach in advance. The detector can control the movement of the magnet in the capsule gastroscope magnetic control system through the position control device on the plane forming an angle of 90 ± 30 degrees with the horizontal plane. The magnetic line direction adjustment device controls the movement of the capsule gastroscope in the stomach of the subject to obtain images of the stomach. By designing the magnetic control system with an examination control mode of upright standing, the cavity of the stomach of the subject can be kept in a relatively complete state, such that the magnetic control system can more easily control the capsule gastroscope. The image efficiency and image effect of capsule gastroscope can be greatly improved.

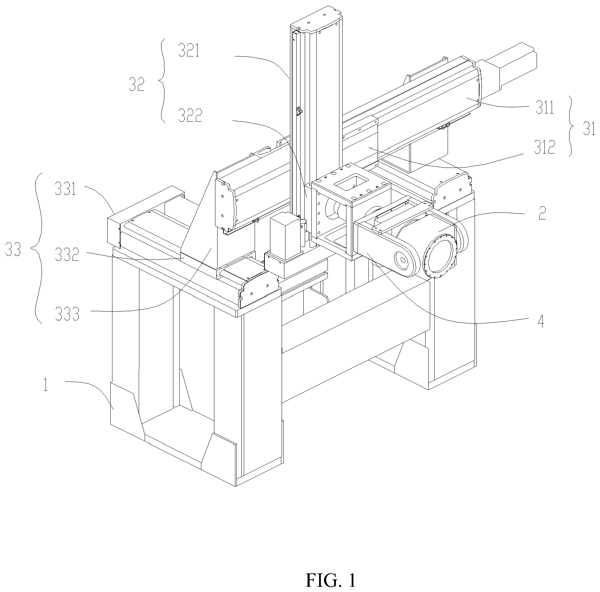

Referring to FIG. 1, a structure of a capsule gastroscope magnetic control system is illustrated, which includes a rack 1 and a magnetic control device located on the rack 1. The magnetic control device includes a magnet 2, a position control device 3 and a magnetic line direction adjusting device 4, which are described in detail as follows:

The position control device 3 is located on the rack 1 which can move the magnet 2 on a plane forming an angle of 90 ± 30 degrees with the horizontal plane.

In the embodiment of the disclosure, the magnet 2 may be a permanent magnet 2 or an electromagnet 2, and the specific shape and size may be adjusted according to an actual demanded performance.

As an embodiment of the disclosure, the position control device 3 includes a first moving portion 31, a second moving portion 32, and a third moving portion 33, specifically.

The first moving portion 31 can make the magnet 2 move along an X axis parallel to the horizontal direction.

In the embodiment of the disclosure, the first moving portion 31 includes a first guide rail 311 located on the rack 1 and the first guide rail 311 is arranged in a direction parallel to the horizontal direction; a first slider 312 capable of sliding along the first guide rail 311; and a first driving module connected to the first slider 312 and configured to drive and control the first slider 312 to slide.

The second moving portion 32 includes a second guide rail 321 located on the first slider 312, and the second guide rail 321 is arranged in a direction perpendicular to the first guide rail 311; a second slider 322 capable of sliding along the second guide rail 321; and a second driving module connected to the second slider 322 and configured to drive and control the second slider 322 to slide; and the magnetic line direction adjusting device 4 is located on the second slider 322.

Through the cooperation of the first moving portion 31 and the second moving portion 32, the magnet 2 located on the magnetic line direction adjustment device 4 can be moved on a plane defined by the X axis and the Y axis by adjustment of the first moving portion 31 and the second moving portion 32. The plane forms an angle of 90 ± 30 degrees with the horizontal plane.

As an embodiment of the disclosure, the plane forms an angle of 90 degrees with the horizontal plane and is relatively parallel to the upright human body to facilitate the magnet 2 to move down to a desired position when the subject is in a upright standing state, so as to control the capsule gastroscope in the subject.

In the embodiment of the disclosure, a third moving portion 33 is further located between the first guide rail 311 and the rack 1, and the third moving portion 33 can make the magnet 2 move along the Z axis which is perpendicular to the plane defined by the X axis and the Y axis. It is understood that the third moving portion 33 may also be moved only along a horizontal direction perpendicular to the X axis.

In the embodiment of the disclosure, the third moving portion 33 includes: a third guide rail 331 located at both ends of the rack 1; a third slider 332 connected to the second guide rail 321 through a supporting member 333, such that the second guide rail 321 is capable of sliding along the third guide rail 331. Specifically, referring to FIG. 1, there are two supporting members 333, one end of the supporting member is fixed to the third slider 332, and the other end thereof is connected to the second guide rail 321, such that the second guide rail 321 is perpendicular to the third guide rail 331. The third slider 332 may be manually moved or mechanically driven to move on the third guide rail 331. The third moving portion 33 is capable of making the magnet 2 move on the magnetic control system along a direction perpendicular to the upright human body to adjust the relative linear distance between the magnet 2 and the human body to achieve the effect of adjusting and controlling the capsule gastroscope.

As an embodiment of the disclosure, the first driving module includes a first driving motor, and a first screw rod driven by the first driving motor to control the first slider 312 to slide. The second driving module includes a second driving motor, and a second screw rod driven by the second driving motor to control the second slider 322 to slide. Of course, in addition to utilize the screw rod drive, it is also possible to use a crawler belt, transmission gear and other transmission methods to drive the slider.

Meanwhile, it is understood that in the embodiment of the disclosure, the position control device 3 realizes the three-axial position adjustment of the magnet 2 through the first moving portion 31, the second moving portion 32, and the third moving portion 33 connected to each other, which is only an optional embodiment of the disclosure, and the positional relationships between of them are interchangeable as long as the three-axial movement control of the magnet 2 can be realized. In addition, the position control device 3 may retain only the X axis and Y axis position control, or may adopt other structures that can achieve similar effects, such as a multi-axis robot, etc., which is not limited in the embodiment of the disclosure.

In the embodiment of the disclosure, the position control device 3 adopts a control approach of three-axis movement control composed of the first moving portion 31, the second moving portion 32, and the third moving portion 33. The position of the magnetic line direction adjusting device 4 provided with the magnet 2 is adjusted to realize the movement control of the magnet 2 on the plane forming an angle of 90 ± 30 degrees with the horizontal plane. In addition, the magnetic control action of the upright standing capsule gastroscope can be completed in cooperation with the standing subject, which has the beneficial effect of high control flexibility and simple structure.

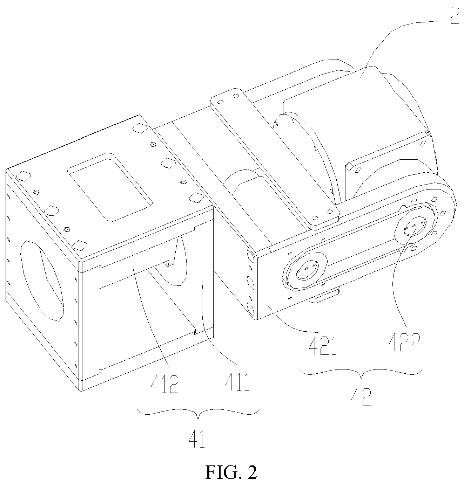

Referring to FIG. 2, a structure of the magnetic line direction adjusting device 4 is shown, which includes a first rotating portion 41 and a second rotating portion 42, which are described in detail as follows:

The first rotating portion 41 has one end connected to the position control device 3 to make the magnet 2 rotate round a first rotational axis direction perpendicular to the X axis and parallel to the horizontal direction.

In the embodiment of the disclosure, the first rotating portion 41 includes a first bracket 411 connected to the position control device, and a fourth driving module 422 located on the first bracket 411 and configured to drive the second rotating portion 42 to rotate round the first rotating axis.

It is understood that the shape of the first bracket 411 is not limited to the embodiments of the disclosure, and may be any shape that functions as a support between the position control device and the second rotating portion 42.

In the embodiments of the disclosure, a third driving module 412 includes a third driving motor, and an end of the rotor of the third driving motor is connected to the second rotating portion 42 and configured to drive the second rotating portion 42 to rotate. Referring to FIG. 1, when the plane where the first guide rail 311 is located is perpendicular to the horizontal plane, the first rotating portion 41 connected to the first slider 312 is perpendicular to a plane defined by the X axis and the Y axis, and is parallel with the horizontal plane. Referring to FIG. 2, specifically, the third driving motor is located in the middle of the first bracket 411, such that the second rotating portion 42 connected thereto rotates in the rotating direction of the motor, which is simple in design and can reduce the volume and cost of the transmission structure. It should be noted that, in practice, the first rotating portion 41 may be driven directly by a motor, and the second rotating portion 42 may be driven by means of a crawler belt driven by a motor, an indirect transmission of a gear set, and the like, which is not limited in the embodiments of the disclosure.

The second rotating portion 42 has one end connected to the magnet 2 and the other end connected to the first rotating portion 41, and can make the magnet 2 rotate round a second rotation axis perpendicular to the first rotation axis.

In the embodiments of the disclosure, one end of the second rotating portion 42 is connected to the magnet 2 through a rotating shaft, and the other end is connected to a second bracket 421 of the first rotating portion 41.

The fourth driving module 422 is located on the second bracket 421 and configured to drive the magnet 2 to rotate round the second rotation axis.

It is understood that the shape of the second bracket 421 is not limited to the embodiment of the disclosure, and may be any shape that functions as a support between the magnet 2 and the first rotating portion 41.

In the embodiment of the disclosure, the fourth driving module 422 located on the second bracket 421 adopts a motor arranged on one side of the magnet 2 to drive the crawler belt connecting the motor and the magnet 2 on one side of the second bracket 421. The magnet 2 is rotationally controlled in the second rotational axis direction such that the magnet 2 is rotated in the second rotational axis direction perpendicular to the first rotational axis direction. Because the first rotation axis is axially parallel with the horizontal plane and perpendicular to the X axis and the second rotation axis is perpendicular to the first rotation axis, such that the second rotation axis rotates axially in a plane substantially perpendicular with the horizontal plane. The plane where the second rotation axis located is parallel to the upright-standing subject.

It should be noted that, in practice, in addition to the driving mode provided in the embodiments of the disclosure that the motor drives the crawler belt to drive the magnet 2 to rotate, the transmission control of the second rotating portion 42 may be directly driven by a motor, a gear set, and the like, which is not limited in the embodiments of the disclosure.

As an embodiment of the disclosure, reducers are provided between the first bracket 411 and the third driving module 412, and between the second bracket 421 and the fourth driving module 422. The reducer not only functions as a bearing, but also enables the magnetic control system to more accurately control the rotational operations of the first rotating portion 41 and the second rotating portion 42.

In practice, the second rotation axis, the first rotation axis, and the three axes in the position control device 3 shown in FIG. 1 cooperatively form a five-axis adjustment system. The system can satisfy the needs of the capsule gastroscope magnetic control system for multi-angle adjustment control of the capsule gastroscope in the stomach of the subject without adjusting the body position too much. Meanwhile, the driving module in each position can adjust the position of the magnet 2 according to a manual input instruction or even automatically according to a program by setting up data processing and position control modules in the magnetic control system, the control efficiency of the capsule gastroscope may be improved, and the time required for the capsule gastroscope to image is greatly reduced.

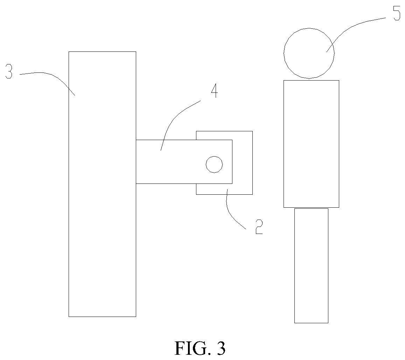

Referring to FIG. 3, in the embodiments of the disclosure, the subject 5 stands in front of the magnetic control system after swallowing the capsule gastroscope, and the magnetic control system adjusts the position and posture of the magnet 2 through the position control device 3 and the magnetic line direction adjusting device 4. Thus, the capsule gastroscope in the subject 5 is pulled to adjust the position and posture correspondingly. Referring to the coordinate axes in FIG. 1, the magnet 2 of the magnetic control system may realize a left and right movement along the X axis in the horizontal direction through a first moving portion or a up and down movement along the Y axis in the vertical or slightly oblique direction through a second moving portion, or a back and forth movement along the Z axis in the horizontal direction through a third moving portion. The movement of the magnet 2 along the X, Y, Z axes can be programmed and controlled by a servo motor to adjust the position of the magnet 2 of the magnetic control system. In addition to this, the magnet 2 of the magnetic control system may also realize a rotational movement in the first rotational axis by the first rotating portion or a rotational movement in the second rotational axis by the second rotational portion. The rotational movement round both the first and second rotational axes may be controlled by a programmable servo motor. The rotational movement of the magnet 2 of the magnetic control system may change the orientation of the magnetic field of the magnet, which drives the adjustments of the corresponding orientation of the capsule gastroscope body, thereby changing the photographing angle of the capsule gastroscope in the stomach, such that the capsule gastroscope camera device can realize photographing at any angle and position, and a comprehensive inspection is achieved in the stomach of the subject 5.

In summary, in the embodiment of the disclosure, the subject stands upright in front of the capsule gastroscope system after swallowing the capsule gastroscope with the camera and the illumination device into the stomach in advance. The detector can control the movement of the magnet in the capsule gastroscope magnetic control system through the position control device on the plane forming an angle of 90 ± 30 degrees with the horizontal plane. The magnetic line direction adjustment device controls the movement of the capsule gastroscope in the stomach of the subject to obtain images of the stomach. By designing the magnetic control system with an examination control mode of upright standing, the cavity of the stomach of the subject can be kept in a relatively complete state, such that the magnetic control system can more easily control the capsule gastroscope. The image efficiency and image effect of capsule gastroscope can be greatly improved.

The foregoing is merely alternative embodiments of the present disclosure and is not intended to limit the present disclosure. Any modifications, equivalent replacements, and improvements made within the ideas and principles of the present disclosure should be considered within the scope of the present disclosure.

## Claims

1. A capsule gastroscope magnetic control system, comprising:
a rack; and
a magnetic control device located on the rack, the magnetic control device comprising:
a magnet;
a position control device located on the rack and capable of moving the magnet on a plane forming an angle of 90 ± 30 degrees with a horizontal plane; and
a magnetic line direction adjusting device located on the position control device and configured to adjust a magnetic line direction of the magnet.

2. The capsule gastroscope magnetic control system of claim 1, wherein the position control device comprises:
a first moving portion capable of moving the magnet along an X axis parallel to the horizontal direction; and
a second moving portion slidably connected to the first moving portion and capable of moving the magnet along a Y axis perpendicular to the X axis;
wherein a plane defined by the X axis and the Y axis forms an angle of 90 ± 30 degrees with the horizontal plane.

3. The capsule gastroscope magnetic control system of claim 2, wherein the plane defined by the X axis and the Y axis forms an angle of 90 degrees with the horizontal plane.

4. The capsule gastroscope magnetic control system of claim 2, wherein the first moving portion comprises:
a first guide rail located on the rack, wherein the guide rail is arranged in a direction parallel to the horizontal direction;
a first slider capable of sliding along the first guide rail; and
a first driving module connected to the first slider and configured to drive and control the first slider to slide.

5. The capsule gastroscope magnetic control system of claim 4, wherein the second moving portion comprises:
a second guide rail located on the first slider, wherein the guide rail is arranged in a direction perpendicular to the first guide rail;
a second slider capable of sliding along the second guide rail; and
a second driving module connected to the second slider and configured to drive and control the second slider to slide;
wherein the magnetic line direction adjusting device is located on the second slider.

6. The capsule gastroscope magnetic control system of claim 5, wherein the first driving module comprises:
a first driving motor; and
a first screw rod driven by the first driving motor and configured to control the first slider to slide.

7. The capsule gastroscope magnetic control system of claim 5, wherein the second driving module comprises:
a second driving motor; and
a second screw rod driven by the second driving motor and configured to control the second slider to slide.

8. The capsule gastroscope magnetic control system of claim 2, wherein the position control device further comprises:
a third moving portion capable of moving the magnet along a Z axis perpendicular to the plane defined by the X axis and the Y axis, or along a horizontal direction perpendicular to the X axis.

9. The capsule gastroscope magnetic control system of claim 8, wherein the third moving portion comprises:
a third guide rail located at both ends of the rack; and
a third slider connected to the second guide rail by a support member, such that the second guide rail is capable of sliding along the third guide rail.

10. The capsule gastroscope magnetic control system of claim 9, wherein one end of the support member is fixed to the third slider, and the other end thereof is connected to the second guide rail, such that the second guide rail is perpendicular to the third guide rail.

11. The capsule gastroscope magnetic control system of claim 1, wherein the magnetic line direction adjusting device comprises:
a first rotating portion having one end connected to the position control device and configured to rotate the magnet round a first rotational axis direction perpendicular to the X axis direction and parallel to the horizontal direction; and
a second rotating portion having one end connected to the magnet and the other end connected to the first rotating portion and configured to rotate the magnet round a second rotation axis perpendicular to the first rotation axis.

12. The capsule gastroscope magnetic control system of claim 11, wherein the first rotating portion comprises:
a first bracket connected to the position control device; and
a third driving module located on the first bracket and configured to drive the second rotating portion to rotate round the first rotating axis.

13. The capsule gastroscope magnetic control system of claim 12, wherein the third driving module comprises:
a third driving motor, wherein an end of a rotor of the third driving motor is connected to the second rotating portion to drive the second rotating portion to rotate.

14. The capsule gastroscope magnetic control system of claim 11, wherein the second rotating portion comprises:
a second bracket having one end connected to the magnet through a rotating shaft and the other end connected to the first rotating portion; and
a fourth driving module located on the second bracket and configured to drive the magnet to rotate round the second rotation axis.

15. The capsule gastroscope magnetic control system of claim 14, wherein reducers are provided between the first bracket and the third driving module, and between the second bracket and the fourth driving module.
